# DEMANDE DE BREVET EUROPEEN

(11) **EP 0 525 714 A1**
(43) Date de publication de la demande: **03.02.1993**
(21) Numéro de dépôt: 92112845.0
(22) Date de dépôt: 28.07.1992
(51) Int. Cl.: A61B 5/11

(54) **Système d'analyse du mouvement d'un objet**

(30) Priorité: 31.07.1991 FR 9109750
(71) Demandeur: ALCATEL ESPACE, F-92407 Courbevoie Cédex (FR)
(72) Inventeur: Hayard, Michel, F-31000 Toulouse (FR); Anne, Jean-Claude, F-31320 Auzeville (FR)
(74) Mandataire: Weinmiller, Jürgen

(57) **Abrégé**

La présente invention se rapporte à un système d'analyse du mouvement d'un objet comprenant un dispositif de transmission de données (14) et au moins un marqueur actif (10) disposé sur ledit corps, permettant une mise en mémoire de mesures de la position dudit marqueur en attendant de pouvoir transmettre celles-ci vers ledit dispositif de transmission de données.

Application notamment au domaine spatial

## Description

La présente invention concerne un système d'analyse du mouvement d'un objet, notamment dans le domaine spatial.

Un tel système, quelquefois appelé "kinésigraphe", est destiné à l'enregistrement des mouvements d'un objet, éventuellement du corps humain. Pour réaliser cette fonction, on positionne sur l'objet observé, des repères visibles que l'on nomme "marqueurs". Le kinésigraphe, qui se compose généralement d'un système de prise de vues et d'un ensemble d'analyse et de traitement d'image, permet alors de filmer ces marqueurs qui portent des marques spécifiques, et en retrouvant ces marques dans l'image, de reconstruire le mouvement étudié. Une telle reconstruction du mouvement peut se faire en deux ou trois dimensions, suivant le besoin.

L'acquisition des informations se faisant généralement de façon optique, il arrive qu'au cours des prises de vues des marqueurs soit cachés ; par exemple dans le cas du corps humain, cachés par d'autres parties du corps. Il est alors difficile, voire impossible, de reconstituer le mouvement correspondant à ces marqueurs cachés. De plus des mouvements de certains objets sont très rapides, comme, par exemple dans le cas du corps humain : les rotations de la tête (360 ° par seconde) ou les mouvements de rotation des chevilles et des poignets. Dans ces cas de mouvements à vitesse rapide, il convient de faire des prises de vues accélérées à des vitesses d'au moins 300 à 400 Hz ; ce qui est généralement impossible du fait des caractéristiques du matériel de prise de vue.

Un certain nombre de systèmes de ce type sont commercialisés. Ils font appel à des techniques d'acquisition variées. Certains font appel à une acquisition d'image de type télévision : tel l'analyseur de mouvement "Elite" commercialisé par la société BTS (Bioengineering Technology and Systems).

Il existe d'autres analyseurs de mouvements qui fonctionnent à l'aide de capteurs PSD ("Position Sensitive Device"). Ils sont rapides (10 KHz) mais aucune image de la scène n'est formée ce qui empêche tout contrôle.

Tous ces systèmes sont incapables d'acquérir des informations à partir d'un marqueur caché, puisque l'analyse et l'élaboration des mesures se fait à partir de l'image. De plus, leur vitesse d'acquisition est limitée (200 Hz maximum).

L'invention a pour objet de concevoir un système permettant d'acquérir les informations des marqueurs animés de grandes vitesses (300 Hz ou plus) et de continuer à acquérir les informations en provenance de marqueurs cachés, de manière à permettre, notamment, d'enregistrer et d'étudier les mouvements d'astronautes en apesanteur.

L'invention propose, à cet effet un système d'analyse du mouvement d'un objet, caractérisé en ce qu'il comprend un dispositif de transmission de données et au moins un marqueur actif disposé sur ledit corps, permettant une mise en mémoire des mesures de position dudit marqueur en attendant de pouvoir les transmettre vers ledit dispositif de transmission de données.

Dans une réalisation avantageuse chaque marqueur actif comprend :
- un capteur de position suivi d'un circuit d'élaboration de mesures de position ;
- une mémoire ;
- des circuits d'émission et de réception de données ;
- un circuit de décision de la cadence des mesures.

Avantageusement ledit système comprend, en outre, au moins une mire disposée sur ledit objet associée à chaque marqueur actif, et un dispositif de prise de vue suivi d'un circuit de traitement de l'image obtenue. Ces mires peuvent avantageusement, être des mires de type code-à-barre circulaire.

Avec un tel système la possibilité d'acquérir des points de mesure sur des marqueurs hors visibilité est évidente.

Pour cinq minutes de mise en mémoire de points de mesure, la taille de la mémoire contenue dans chaque marqueur actif n'excède pas 1 Méga- bit, en considérant une mesure en moyenne tous les 1/50ème de seconde et 64 bits par point. Dans le cas de mouvements rapides, la même taille mémoire correspond à moins d'autonomie (beaucoup plus de points de mesure dans le même temps). De plus, la vitesse d'acquisition n'étant pas liée aux cadences de prise de vue des caméras mais fonction de la rapidité du mouvement, des vitesses supérieures à 300 Hz peuvent être utilisées pour l'élaboration des points de mesure. Enfin, le choix automatique de la vitesse de mesure par les marqueurs, en fonction de la rapidité du mouvement, permet "de facto" une compression d'information exactement adaptée au besoin.

Les caractéristiques et avantages de l'invention ressortiront d'ailleurs de la description qui va suivre, à titre d'exemple non limitatif, en référence aux figures annexées sur lesquelles :
- la figure 1 illustre le fonctionnement du système de l'invention ;
- la figure 2 représente les différents circuits qui forment un marqueur actif selon l'invention.

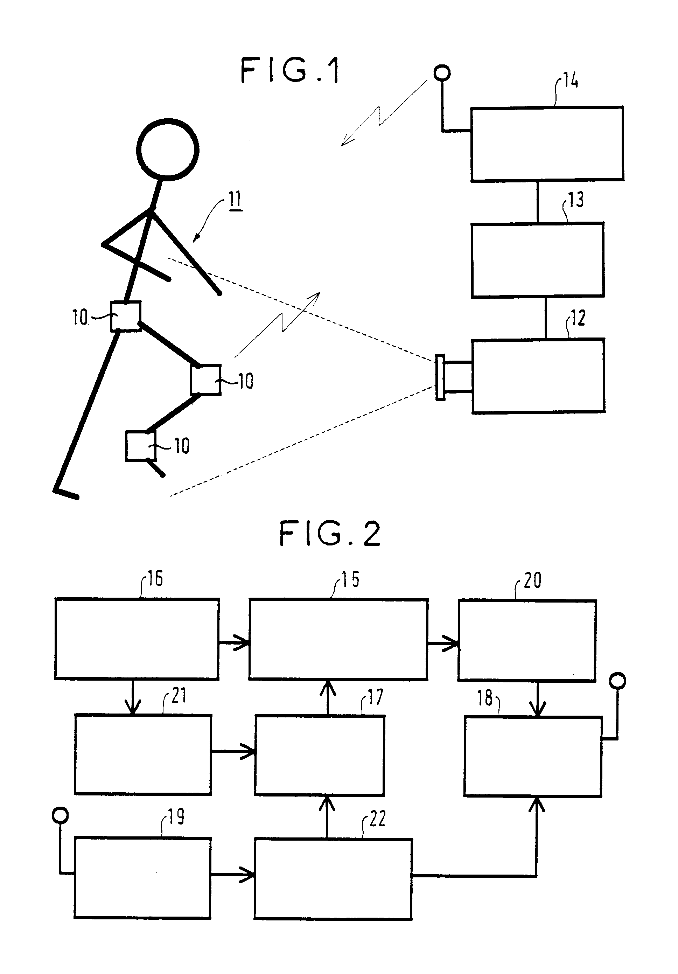

Le système de l'invention, tel que représenté sur la figure 1, comprend :
- des marqueurs 10 disposés sur le corps 11 en mouvement,
- des dispositifs de prise de vue 12 et de traitement 13 de l'image obtenue,
- un dispositif de transmission de données 14.

Les marqueurs 10 sont des marqueurs actifs, qui sont associés à des mires passives comme par exemple des mires de type code-barre circulaire.

Chaque marqueur actif comprend :
- un capteur de position 16 suivi d'un circuit 15 d'élaboration des mesures,
- une horloge 17,
- des circuits d'émission 18 et réception 19 de données par exemple dans le domaine infra-rouge ; le circuit de réception 19 étant suivi d'un circuit de traitement 22 permettant le décodage des informations reçues, la mise à l'heure et la calibration,
- une mémoire numérique 20 de taille suffisante,
- des circuits de décision 21 qui sélectionnent la cadence d'échantillonnage des mesures en fonction de l'accélération instantanée du marqueur.

Une forte accélération, indiquant un mouvement brusque et rapide, nécessite un plus grand nombre de points de mesure par seconde qu'un mouvement lent. Dans ce cas, le marqueur augmente de lui-même (automatiquement), la cadence d'échantillonnage jusque vers 300 ou 400 Hz, ou plus si nécessaire, et ceci uniquement pendant le mouvement rapide. Quand le mouvement redevient lent, la cadence d'échantillonnage des mesures revient à 50 Hz ou moins. Tous ces points de mesure sont datés et mis en mémoire dans la mémoire 20 du marqueur, avant de pouvoir être retransmis.

Le dispositif de prise de vue 12 se compose d'une caméra (ou de plusieurs si l'on veut des mesures en trois dimensions) fixe, associée à un circuit de traitement d'image 13 effectuant les opérations suivantes :
- détection de code, par exemple de code-barre ,sur le signal vidéo, dans le cas d'une mire de type code-barre associée au marqueur actif. Cette détection doit pouvoir se faire de façon asynchrone : c'est justement une caractéristique des code-barres qui comportent aussi leur propre horloge de décodage. On s'affranchit ainsi des variations de distance de prise de vues qui rendent la lecture du code plus ou moins longue.
- détermination des instants de décodage, ce qui permet de déterminer la position du centre du marqueur. Le numéro de la ligne, où se fait le décodage (ou le numéro de la ligne centrale de plusieurs décodages successifs), indique la valeur "y" de la position du marqueur par rapport à la caméra. L'instant de décodage sur cette ligne indiquant la valeur "x" de cette position.

Le dispositif de transmission de données 14 se compose d'un moyen de synchronisation des marqueurs (par exemple un flash infra-rouge à chaque trame TV), d'un moyen d'adressage des marqueurs permettant de les interroger (par exemple le numéro du marqueur envoyé en même temps que le flash de synchronisation), et d'une réception infra-rouge (ou autre) permettant de récupérer les informations contenues dans les marqueurs.

Un protocole d'échange et de communication est à définir avec précision. Il en existe plusieurs qui sont connus de l'homme de l'art. Un tel protocole doit cependant être capable de :
- interroger séquentiellement tous les marqueurs en les adressant sans ambiguïté,
- interroger seulement ceux qui sont visibles par la caméra ; c'est-à-dire ceux dont le code peut être décodé,
- transmettre périodiquement une mise à l'heure à tous les marqueurs,
- mettre en oeuvre un protocole (accusé de réception ou autre méthode) évitant de perdre des données.

Ce système de transmission doit aussi être capable de stocker toutes les données en provenance des marqueurs et aussi celles venant du traitement d'image des caméras.

On va expliciter, à présent le principe de fonctionnement du système de l'invention.

Les mesures sont faites en deux endroits : dans les marqueurs et dans le circuit (ou les circuits) qui est (sont) situés après la (ou les) caméras. C'est la combinaison de ces informations qui donne la mesure finale.

Chaque marqueur génère lui-même, et en interne, ses propres points de mesure, qui sont échantillonnés en fonction de la rapidité du mouvement. Il met ces points en mémoire en attendant (pendant par exemple quelques minutes) d'être interrogé.

Les points de mesure élaborés dans chaque marqueur sont repérés dans un espace à 3 dimensions et datés. Ils peuvent comporter en plus le numéro du marqueur pour identifier l'origine de la mesure ; Mais ceci n'est pas absolument nécessaire puisque l'on sait quel marqueur interrogé est en train de répondre. Ce numéro doit être ajouté, en tous cas, à la réception, pour identifier la provenance du point de mesure : coordonnées x, y, z ; datation t ; identification n.

Quand une caméra "voit" un marqueur, c'est-à-dire décode le code de la mire associée, le traitement d'image élabore un point de mesure par rapport à cette caméra (en deux dimensions, daté et identifié par le numéro du marqueur) : xi, yi, t, n. Le système de transmission peut alors interroger ce marqueur pour vider le contenu de sa mémoire puisqu'il est en "visibilité". On peut alors facilement recaler les points de mesure "relatifs" donnés par chaque marqueur, à l'aide des points de mesure "absolus" donnés par la caméra à partir de la mire associée à ce marqueur "n".

Lors de la mise en route on réalise une procédure d'étalonnage du système, qui définit les positions initiales et la date initiale. Elle peut se faire au début de l'expérience, par exemple par la prise de vue d'un corps de référence équipé de marqueurs, et filmé sans mouvement dans différentes positions.

Le système de l'invention doit disposer d'une grande mémoire de stockage de tous les points de mesure pour un traitement ultérieur, si besoin est.

Il est bien entendu que la présente invention n'a été décrite et représentée qu'à titre d'exemple préférentiel et que l'on pourra remplacer ses éléments constitutifs par des éléments équivalents sans, pour autant, sortir du cadre de l'invention.

Plusieurs variantes du dispositif de l'invention sont possibles :
- Dans une première variante avec deux caméras, ou plus, il est possible de réaliser les mêmes opérations dans un espace à trois dimensions. Des circuits électroniques sont alors ajoutés pour calculer les points de mesure absolus (à 3 dimensions) : x', y', z', t, n, à partir des points de mesure issus des caméras (à deux dimensions : x1, y1, t, n : caméra 1, et x2, y2, t, n : caméra 2, ce qui permet de recaler les points de mesure "relatifs" donnés par les marqueurs.
- Dans une seconde variante on utilise un moyen de transmission différent de l'infra-rouge avec les marqueurs fonctionnant par exemple en :
- radio fréquence,
- optique (laser).
- Dans une troisième variante possible on utilise des mires, autres que des mires circulaires de type code-barre, telles que :
- des mires codées en couleur,
- des mires "clignotantes",
- des mires à dessin spéciaux,
- des mires de type code-barre classique (droite).

## Revendications

1. Système d'analyse du mouvement d'un objet, caractérisé en ce qu'il comprend un dispositif de transmission de données (14), et au moins un marqueur actif (10) disposé sur ledit objet, permettant une mise en mémoire des mesures de position dudit marqueur en attendant de pouvoir les transmettre au dispositif de transmission de données.

2. Système selon la revendication 1, caractérisé en ce que chaque marqueur actif (10) comprend :
- un capteur de position (16) suivi d'un circuit (15) d'élaboration des mesures ;
- des circuits d'émission (18) et de réception (19) des données ;
- une mémoire (20) ;
- un circuit de décision (21) de la cadence des mesures.

3. Système selon l'une quelconque des revendications 1 ou 2, caractérisé en ce qu'il comprend un dispositif de prise de vue suivi d'un circuit de traitement de l'image obtenue (13), et au moins une mire, disposée sur ledit objet, associée à chaque marqueur actif.

4. Système selon la revendication 1, caractérisé en ce que chaque mire est une mire de type code-barre circulaire.

5. Système selon l'une quelconque des revendications précédentes, caractérisé en ce qu'il est utilisé pour étudier le mouvement du corps humain.

6. Système selon la revendication 1 caractérisé en ce qu'il comprend un dispositif de réception de données faisant partie dudit dispositif (14) de transmission de données.
